# EUROPEAN PATENT APPLICATION

(11) **EP 2 351 556 A2**
(43) Date of publication of application: **03.08.2011**
(21) Application number: 09823867.8
(22) Date of filing: 30.10.2009
(51) Int. Cl.: A61K 9/54, A61K 9/16, A61P 7/04, A61K 47/34, A61K 9/127, A61K 9/00

(54) **ENCAPSULATED FUNCTIONAL FINE PARTICLE COMPOSITION CAPABLE OF SPRAYING AND PREPARATION METHOD THEREOF**

(30) Priority: 31.10.2008 KR 20080108042
(71) Applicant: Genewel Co., Ltd, Gyeonggi-do 462-807 (KR)
(72) Inventor: CHOI, Jin-suk, Seoul 135-090 (KR); LEE, Young-woo, Suwon-si Gyeonggi-do 440-300 (KR); KIM, Jun-ho, Incheon 402-200 (KR); SHIM, Eun-young, Seongnam-si Gyeonggi-do 462-724 (KR)
(74) Representative: Golding, Louise Ann
(86) International application number: PCT/KR2009/006365
(87) International publication number: WO 2010/050787

(57) **Abstract**

Provided is an encapsulated functional fine particle composition capable of spraying that is useful for hemostasis and wound protection and allows a patient to treat the wound by oneself. Additionally, the composition can be rapidly applied on a large wound during an operation using an air gun in case of in-vivo application and shows prompt hemostasis.

## Description

### BACKGROUND OF THE INVENTION

### Field of the Invention

The present invention relates to an encapsulated functional fine particle composition capable of spraying and preparation method thereof; and, more particularly, to an encapsulated functional fine particle composition capable of spraying and preparation method thereof characterized in that comprises double layers which includes an outer layer consisting of biodegradable, biocompatible natural polymer and an interior layer consisting of heat sensitive functional synthetic polymer capable of supporting with drugs and uses a volatile solvent or gas as a carrier.

### Description of Related Art

A hemostasis is an important factor for deciding the life of the patient within 24hours after the wounds are occurred. After the fatal wounds are occurred, the average arrival time to the hospital of the patient is about 20mins, this is the time that the possibility of dying with hypotension is 50%. Further, in the case of normal adult, if more than 10% of weight is lost by bleeding, it is necessary to do a prompt hemostatic treatment because it begins to be dangerous by going into shock which the whole tissues in the body becomes the hypoxic state. Accordingly, the success of the effective hemostasis acts as an important factor such as decreasing of the possibility for dying and secondary infection at occurring exterior wound, minimizing the blood loss during an operation, decreasing post-operative complications, shortening the operation time, etc. However, general pressure dressing, bandage, specific point compression are effective against a hemostasis, it is difficult to effectively easily stop the bleeding quickly.

Effectiveness and stability are the most important for a hemostatic, it has excellent hemostatic effectiveness, it must be stable during the tissue reaction and cure procedure in the body, and have to be completely degraded and absorbed when being remained in the internal organs. Further, it has to be convenient to apply for user and be inexpensive. However, most of the existing biodegradable products for suppressing bleeding contains a protein agent, so it must be stored at a low temperature, and has disadvantages of not easily adhering to the affected areas like organs and tissues in the body owing to the structural property where most of the type is sponge or glue and the difficulty in description. In addition, most of the external hemostatics are band-aid type therefore has low hemostasis, hemostasis speed is not fast, and spread type of hemostatic previously developed is difficult to be used for hemostatic because it is made up of oxycellulose thereby causes the foreign-object and inflammatory reaction, has a problem that it acts as simple hemostatic mechanism therefore requires additional aid for protecting wound or preventing adhesion.

An adhesion of organs and tissues occurring after an operation is a physiological phenomenon which occurs in the course of proliferation and regeneration of cells in the wounded tissue. However, excessive adhesion of tissues or adhesion to the other tissue and organ unintended causes disorder to the functions of organs or tissues, therefore requires secondary operation for synechotomy, or threat of life in some cases. The adhesion of the tissue after operation occurs in most parts of the human body, particularly as causes of organ adhesion after abdominal operation, foreign substances flowed into an abdominal cavity, inflammatory reaction caused by infection, ischemia of tissue, blood coagulation, rupture of serous membrane, etc., however until now there is no definite method of preventing them clinically. The possibility of adhesion after bleeding is very high, however a hemostatic having the preventing effect adhesion as well as bleeding is not yet developed. When using a hemostatic gauze in the form of fabric made of oxycellulose, the inflammatory reaction may be caused or the adhesion may be further promoted by emitting acidic degraded material in case of being degraded. Accordingly, there remains to develop functional hemostatic which is correctly apply to the large irregular affected area, forms hemostatic mechanism, and may be anticipated the effects of hemostatic, protection of wounded, or prevention of adhesion.

A material for a hemostatic being currently used includes biocompatible natural polymer including polysaccharides, non-biocompatible natural polymer, etc. Concretely, oxidized cellulose(OC), methyl cellulose, ethyl cellulose, hydroxyethyl cellulose, calcium alginate, dextran sulfate, sodium hyaluronate(HA), chondroitin sulfate(CS) and collagen, fibrin, gelatin, etc. are useful. These materials are used as alone or with forming a specific structure together.

US Patent No.6,706,690 which is a prior art regarding to such hemostatic, discloses the dried blood-active composition comprising a biocompatible polymer be crosslinked with gelatin as raw material, and uncrosslinked biocompatible polymer, wherein the crosslinked polymer becomes hydrogel when being exposed to blood, the uncrosslinked polymer relatively promptly melt, so that it may be used as hemostatic or drug carrier.

In addition, US Patent No.7,262,181 discloses a hemostatic material comprising water-soluble cellulose ether derivative such as methyl cellulose, ethyl cellulose, hydroxyethyl cellulose and carboxymethyl cellulose in the form of medically useful salt, and particularly bio-absorptive water-soluble cellulose ether derivative, wherein the structure thereof is the form of fiber, fabric, felt, sponge, film, capsule, column, colloid.

US Patent No.6,432,415 discloses a composition which non-water-soluble alkyl cellulose and volatile solvent and water, dissolving agent and dispersant are mixed, and gel or aerosol is applied as a carrier for sending a lot of drugs having various solubility into specific part.

US Patent No. 6,372,196 discloses a system comprising the fine dispersed polyanhydroglucoronic acid and/or salts thereof propellant, as an aerosol for promptly stopping bleeding of various wounds by a stab, bums, a cut, etc. and preventing secondary infection, and the use for a hemostatic. WO2006/006140A1 discloses a composition of glycerol plasticizer material and pectin that is bioadhesive polymer, this is dispersed into the wounds by comprising a fine dispersed oxycellulose, peripherally treated to the surface of scaly wounds such as the wounds from shaving or small stab.

However, a hemostatic having the form of such hydrogel, fiber, foam, felt, etc. have a difficulty of applying on the wounds part promptly accurately, and revealing of effectiveness by risk of infection by contacting with medical team when treating. In addition, an oxycellulose-based hemostatic has lower biocompatibility than other bio-derived material because it is not bio-derived material, and high possibility of inflammation because it represents acidic pH when being degraded, and promptly denatured when being contacted with acid-sensitive hemostatic protein including thrombin or fibrinogen. Further, if the type of the product is aerosol, it is able to easily apply to large parts, however a role such as a hemostatic, a protection of the wounds, etc. for curing the wounds cannot be anticipated because it has only the hemostasis and cannot be properly fixed and may be flowed down when applying on the wounds. Also, in the case of hemostasis during an operation not being external wounds, a hemostasis and a protection of the wounds as well as an adhesion by the wounds and the blood have to be considered.

Greenplast produced by Green Cross Corp., among the hemostatic products on sale currently, is a product having 70% or more of the domestic market share in hemostatic, and is used by gelation by treating to small affected parts at an operation. However, this product is used by mixing thrombin with fibrin glue, and has disadvantage of keeping refrigerated and difficulty of promptly treating to the large wounds. Meanwhile, Gelfoam in the form of sponge which is a gelatin-based product, is largely used to the hemostasis at an operation, however it has a variety of restrictions that the difficulty of treating to exterior wounds, the risk of infection by hands of surgeons, the minimization the moisture in the operation.

In addition, Surgicel of Johnson & Johnson, Oxicel of BD are a hemostatic gauze in the form of fabric being made from oxycellulose, are mainly used when being hard to suture, as being described above, it may cause the inflammatory reaction by emitting acidic degraded material in case of being degraded in the body, and may be infected by contacting when being applied during an operation.

Seal on, a spray type of hemostatic, of Altracel is a hemostatic made from oxycellulose, is applied on the wounds by using volatile solvent and gas as a carrier, and is easy to apply to the wounds, however it may be applied only to exterior wounds, and has a drawback of flowing down from the wounds when being applied for the hemostasis, and a drawback of not having the effect of hemostasis and disinfection, protection of wounds, except for hemostasis of small cut.

As a result, the above prior arts have to apply to the affected parts by the structure of gel, sponge, fabric, etc., it is difficult to promptly treat, and possible to be contaminated by contacting when applying. In addition, when the used raw material is directly contacted with tissue or blood, it cannot be avoid inflammatory reaction, may be denatured by contacting with hemostatic protein when being degraded, and it has a drawback of requiring the specific storage condition. Further, in the case of a spray type of hemostatic, it is easy to promptly treat, but is difficult to use at an operation, be positioned to the applied wounds part and has a drawback of not having the effect of hemostasis and disinfection, protection of wounds being followed the treatment of the wounds.

A hemostatic has to meet the several requirements for use in accordance with their purpose and use.

Firstly, it has to be promptly accurately applied on a wound for excellent hemostasis, secondly, a foreign body inflammatory reaction has to be minimized because it has to be safe in the body during the response of tissue and healing process. Thirdly, it has to completely be degraded and absorbed when being remained in the internal organs, fourthly, it has to be convenient to apply for general user and doctor, and not to be contaminated by contacting at an operation. In addition, it has to be definitely fixed to the wounds part after treatment, the preferable hemostatic is the hemostatic that it has the effect of hemostasis and protection of the wounds, and the prevention of adhesion after an operation.

The present inventors resolve the above problems of the prior art over the present invention, is able to develop a hemostatic suitable for requirements as hemostatic.

### THE DETAILED DESCRIPTION OF THE INVENTION

### TECHNICAL PROBLEMS

An embodiment of the present invention is directed to providing a functional fine particle composition capable of spraying and the preparation method thereof, which improves availability of treatment and narrowness of the treatment range, the possibility of contamination by contacting which are drawbacks of the gel, solution, sponge, fabric, aerosol types of hemostatic, improves inflammation and foreign response, the possibility of denaturing at degradation, particularity of the storage requirements, etc. which are drawbacks of oxycellulose, gelatin, protein agent-containing hemostatic based hemostatics, capable of improving the use convenience of users together with improving of hemostasis performance.

Another embodiment of the present invention is directed to providing a functional fine particle composition capable of spraying and the preparation method thereof, which has prompt hemostasis and the wounds protection of dressing when applying on the exterior wounds, and prompt hemostasis against large wounds and adhesion prevention against the wounds at an operation, by increasing the hemostasis against the wounds and the protection of adhesion and the wounds against the wounded parts, by which the hemostatic functional component and heat-sensitive functional component have double fine particle structure.

Another embodiment of the present invention is directed to providing a functional fine particle composition capable of spraying and the preparation method thereof, which allows a patient easily to treat the wound by oneself from the body by filling volatile solvent or gas, it may be rapidly applied on a large wound using air gun during an operation, it is simple to operate, and it has a decreasing of the infection possibility by contacting, and effect of hemostasis and adhesion prevention.

To achieve the object of the present invention, the present invention provides a functional fine particle composition capable of spraying, comprising:
a) a core layer of a double-layered fine particle, comprising heat-sensitive functional polymer and capable of supporting with a drug;
a) an outer layer of the double-layered fine particle, surrounding the core layer and comprising biodegradable-biocompatible polymer; and
c) a carrier comprising a volatile solvent or gas carrying the double-layered fine particle.

Further, the present invention provides a method of preparing a functional fine particle composition capable of spraying, comprising:
a) forming a core of double-layered fine article capable of supporting with drugs and comprising heat-sensitive functional polymer; and
b) forming an outer layer of double-layered fine particle comprising biodegradable-biocompatible polymer; and
c) filling a carrier comprising volatile solvent or gas.

Hereinafter, the present invention will be described in detail.

The present inventors made the present invention on the basis of the method comprising forming the double-layered functional fine particle which biocompatible-biodegradable polymer having hemostasis performance is coated to the surface of the functional polymer, filling volatile solvent or gas as carrier, and preparing the functional fine particle composition capable of spraying, and confirmation that it is promptly and easy to treat to the wounded part, shows the prompt hemostasis of the biocompatible-biodegradable polymer having the hemostasis performance, and the wounds protection and adhesion prevention effect remaining to be adhered to the wounds, for heat-sensitive functional polymer.

The hemostatic of the present invention will be described in more detail as follows.

<Double-layered functional fine particle>

The outer layer of the double-layered functional fine particle according to the present invention comprises biodegradable-biocompatible polymer.

The biodegradable-biocompatible polymer includes glycoaminoglycan such as chondroitin sulfate, dermatan sulfate, keratan sulfate, heparan sulfate, hyaluronic acid, alginate and heparin; protein such as collagen, gelatin, elastin and fibrin; proteoglycan such as versican, aglycan, perlecan, decorin, viglican, celgricin and syndecan; glycoprotein such as fibronectin, laminin, vitronectin, thrombospondin and tenascin; phospholipid such as phosphatidylcholine, phosphatidylcerine, phosphatidylethanolamine, spingomierin and the derivative thereof; or glycolipid such as cerebroside, ganglioside, gallactocerebroside and the derivative thereof, and cholesterol.

The outer layer of the fine particle may be crosslinked by a crosslinker, the crosslinker includes a compound containing at least one cation selected from the group consisting of Mg²⁺, Mn²⁺, Ca²⁺, Co²⁺, Cu²⁺, Sr²⁺, Ba²⁺ and Fe²⁺, or an acrylic polymer comprising chitosan, glutaraldehyde, formalin, poly-L-lysine, polyacrylic acid and polymethacrylic acid, a hydroxylamine compound containing dopamine, one or two more selected from amino acids and the polymer thereof comprising isoleucine, phenylalanine, leucine, threonine, lysine, tryptophan, methionine, valline, histidine, alanine, arginine, asparagine, aspartate, cysteine, glutamine, glutamate, glycine, proline, serine and tyrosine.

The core of the double-layered functional fine particle according to the present invention is comprised of one or two more copolymer selected from polyethyleneglycol-polypropyleneglycol copolymer, polyethyleneglycol-polylactic acid copolymer, polyethyleneglycol-polylactic glycolic acid copolymer and polyethyleneglycol-polycaprolactone copolymer, and is able to increase the effect of hemostasis and the wounds protection and adhesion prevention and convenient to use by using the heat-sensitivity and being harmless to humans. The weight average molecular weight is preferably more than 1,000daltons, below 100,000daltons, more preferably more than 5,000daltons, below 50,000daltons.

The core is suitably mixed when being dissolved into the body fluid in the body, or gelated by the body temperature with heat-sensitivity of the copolymer chemically combined, so it may be applied as a barrier of the wounds protection and adhesion prevention with being intensively existed in the wounds of the tissue.

The core may contain the drug, the drug is used by combining one or more selected from the group consisting of thrombin, aprotinin, steroidal anti-inflammatory drug and non-steroidal anti-inflammatory drug. The content of the drug is preferably 0.1 to 40% by weight. If below than 0.1 % by weight, the effect by the drug may weak, and if greater than 40% by weight, it is difficult to be stably mixed into the fine particle.

In the double-layered functional fine particle, a)biodegradable-biocompatible polymer is preferably 20 to 99% by weight, b)heat-sensitive functional polymer is preferably 1 to 80% by weight. If the biodegradable hemostatic polymer is below than 20% by weight, the hemostasis is decreased by not forming blood clot on the wounds or skin surface, and if the heat-sensitive copolymer is mixed in the amount of greater than 80% by weight, it is hard to expect the hemostasis and inconvenient to use by being gelated even at low temperature.

The double-layered functional fine particle is preferably made to the diameter of preferably 0.01 µm to 400 µm, more preferably 0.1 µm to 200 µm. If the double-layered fine particle is so small, biodegradable hemostatic polymer and heat-sensitive copolymer cannot form the fine particle in the appropriate ratio, so it is difficult to properly perform the hemostasis and the wounds protection function, and if the particle is so large, it is difficult to discharge the fine particle when applying by using spray. Accordingly, a hemostatic having hemostasis and the wounds protection while being easy to spray, may be developed only by preparing the fine particle having fixed size through the suitable speed of stirring,

The diameter of the fine particle means the average size of the dried fine particle, and is measured by using SEM(scanning electron microscope) device.

<Volatile carrier>

The volatile carrier be used in the present invention includes liquid version or gas version ethanol, isopropanol, propanol, butanol, 1,1-fluoroethane, propane, butane, nitrogen, air, carbon dioxide, etc.

Preferably, the volatile carrier should be contained in amount of 20 to 90% by weight, more preferably 30 to 80% by weight in the whole composition. If the content of the volatile carrier is less than 20% by weight, or over than 90% by weight, it has problems that the easy to spray, homogeneous mixing, sufficient applying for hemostasis cannot be sufficiently secured.

The hemostatic according to the present invention is easy to treat to the wounds, acts a hemostasis by forming blood clot of biodegradable-biocompatible polymer of the double-layered functional fine particle when being applied on the wounds of exterior body and increases the adhesion to the tissue and wounds of the heat-sensitive copolymer, and the supported drug has a promotive action of the hemostasis, or acts as disinfection and inflammatory reaction, and show the effect of the wounds protection and adhesion prevention. Further, there is a few inflammatory reaction or possibility of remaining which is able to occur when being degraded, because their biocompatibility on the surface of being contacted is excellent.

The preparation method of the double-layered functional fine particle of the present invention includes a)the method of preparing powder by forming an emulsion followed by drying, b)the method of preparing powder by extracting an emulsion to nonsolvent, c)the method of preparing by spray-coating to the powder for core followed by drying, d)the method of preparing powder by mixing the biodegradable hemostatic polymer with heat-sensitive copolymer followed by spray-drying, e)the method of preparing the power by electric spraying, f)the method of preparing fine particle by pulverizing the powder which is mixed with biodegradable hemostatic polymer and heat-sensitive copolymer and dried.

### Effect of Invention

The functional fine particle composition capable of spraying according to the present invention improves availability of treatment and narrowness of the treatment range, the possibility of contamination by contacting which are drawbacks of the gel, solution, sponge, fabric, aerosol types of hemostatic, improves inflammation and foreign response, the possibility of denaturing at degradation, particularity of the storage requirements, etc. which are drawbacks of oxycellulose, gelatin, protein agent-containing hemostatic based hemostatics. Accordingly, it is promptly and easy to treat to the wounded part, and shows the prompt hemostasis of the biocompatible-biodegradable polymer having the hemostasis performance, and the wounds protection and adhesion prevention effect remaining to be adhered to the wounds, for heat-sensitive functional polymer.

### BRIEF DESCRIPTION OF THE DRAWINGS

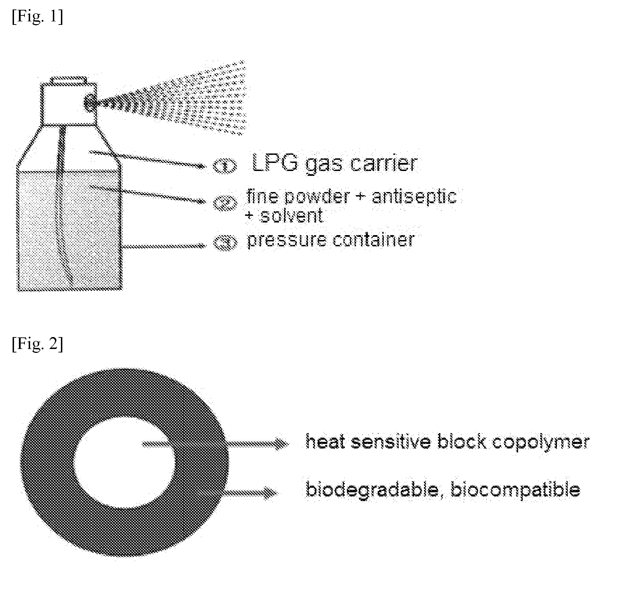

Fig. 1 is a rough diagram of an encapsulated functional fine particle composition product capable of spraying in accordance with an embodiment of the present invention.

Fig. 2 is a scheme of an encapsulated functional fine particle composition product capable of spraying in accordance with an embodiment of the present invention.

Fig. 3 is a photograph of a scanning electron microscope of a drug that is supported to the encapsulated functional fine particle composition product capable of spraying according to example 13 of the present invention.

Fig. 4 is a comparative figure for degradation degree of Surgicel R which is a commercial hemostatic, and a powder hemostatic two weeks after a particle hemostatic(hereinafter, be called as 'powder hemostatic') that is prepared according to the example 13, was treated to SD-rat.

Fig. 5 is a comparative figure for H&E dyeing (x100) of liver, kidney and spleen two weeks after a particle hemostatic(hereinafter, be called as 'powder hemostatic') that is prepared according to the example 13, was treated to SD-rat.

### DESCRIPTION OF SPECIFIC EMBODIMENTS

The advantages, features and aspects of the invention will become apparent from the following description of the embodiments with reference to the accompanying drawings, which is set forth hereinafter.

Examples 1 to 7 <Preparation of a double-layered particle>

As seen in following Table 1, the double-layered fine particle was prepared by varying the type and content of biodegradable-biocompatible polymer, and the content of the heat-sensitive poloxamer selected from polyethyleneglycol-polypropyleneglycol copolymer, polyethyleneglycol-polylactic acid copolymer, polyethyleneglycol-polylactic glycolic acid copolymer and polyethyleneglycol-polycaprolactone copolymer (see following example 13).

**[Table 1]**

| Division | Biodegradable-biocompatible polymer | | Heat-sensitive copolymer(poloxamer) |
|---|---|---|---|
| | kind | Content (% by weight) | Content (% by weight) |
| Example 1 | Keratan sulfate | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 2 | Hyaluronic acid | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 3 | Alginate | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 4 | Gelatin | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 5 | Collagen | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 6 | Fibrin | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |
| Example 7 | Elastin | 20 | 80 |
| | | 30 | 70 |
| | | 40 | 60 |
| | | 50 | 50 |
| | | 60 | 40 |
| | | 70 | 30 |
| | | 80 | 20 |
| | | 90 | 10 |
| | | 99 | 1 |

Examples 8 to 10 <Support of a drug to the heat-sensitive copolymer>

As seen in following Table 2, when preparing the heat-sensitive poloxamer selected from polyethyleneglycol-polypropyleneglycol copolymer, polyethyleneglycol-polylactic acid copolymer, polyethyleneglycol-polylactic glycolic acid copolymer and polyethyleneglycol-polycaprolactone copolymer, it is possible to support(by using the method of adding and mixing drug when preparing the heat-sensitive copolymer solution), and it was prepared by varying the type and content of the supported drug.

**[Table 2]**

| Division | Supported drug | | Heat-sensitive copolymer(poloxamer) |
|---|---|---|---|
| | kind | Content (% by weight) | Content (% by weight) |
| Example 8 | Thrombin | 0.1 | 99.9 |
| | | 1.0 | 99.0 |
| | | 5.0 | 95.0 |
| | | 10.0 | 90.0 |
| | | 20.0 | 80.0 |
| | | 30.0 | 70.0 |
| | | 40.0 | 60.0 |
| Example 9 | Aprotinin | 0.1 | 99.9 |
| | | 1.0 | 99.0 |
| | | 5.0 | 95.0 |
| | | 10.0 | 90.0 |
| | | 20.0 | 80.0 |
| | | 30.0 | 70.0 |
| | | 40.0 | 60.0 |
| Example 10 | Centella asiatica | 0.1 | 99.9 |
| | | 1.0 | 99.0 |
| | | 5.0 | 95.0 |
| | | 10.0 | 90.0 |
| | | 20.0 | 80.0 |
| | | 30.0 | 70.0 |
| | | 40.0 | 60.0 |

As seen in above Table 2, it was easy to prepare and be dispersed it regardless of the content of the drug, however, if the content was low, it was hard to expect strong pharmacological effect.

Examples 11 to 12 < filling of volatile carrier

As seen in following Table 3, when dispersing the encapsulated double-layered fine particle being made from biodegradable-biocompatible polymer and heat-sensitive copolymer into volatile carrier, it was prepared by varying the encapsulated double-layered fine particle and volatile carrier.

**[Table 3]**

| Division | Volatile carrier | | Encapsulated double-layered fine particle |
|---|---|---|---|
| | Kind | Content (% by weight) | Content (% by weight) |
| Example 11 | Ethanol | 20 | 80 |
| | | 40 | 60 |
| | | 70 | 30 |
| Example 12 | 1,1-fluoroethane | 20 | 80 |
| | | 40 | 60 |
| | | 70 | 30 |

As If the content of the volatile carrier is less than 20% by weight, it was not easy to spray and was able to uniformly mix.

Example 13

The heat-sensitive copolymer(poloxamer) was added into n-Hexane solution in the amount of 10% by weight, stirred and formed the emulsion, followed by adding 10% by weight of gelatin solution and again stirred. At this time, the stirring speed was maintained at 4500rpm, so double emulsion was formed. The prepared emulsion solution was stirred while being dropped to excessive ethanol. The extracted particle was centrifuged, and the particle having 50µm to 200µm was separated by using sieve. The separated particle was filled with 70% by weight of liquefied propane gas, to prepare the functional fine particle composition capable of spraying.

Example 14 <Stability in the body Test at SD-rat>

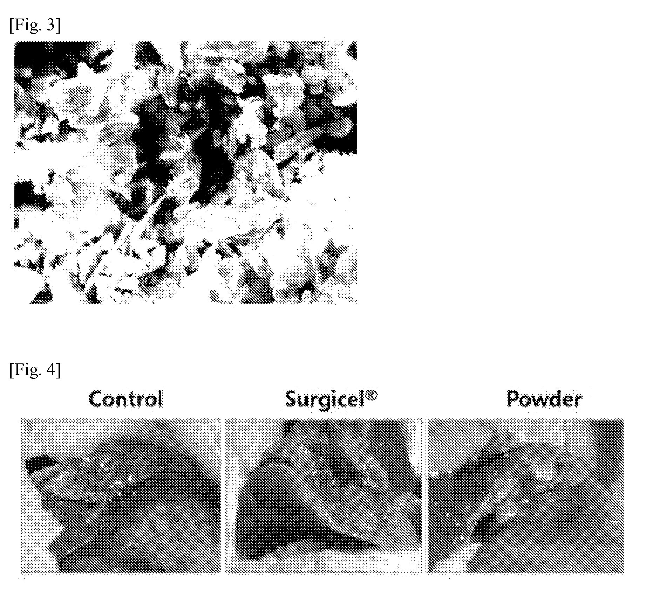

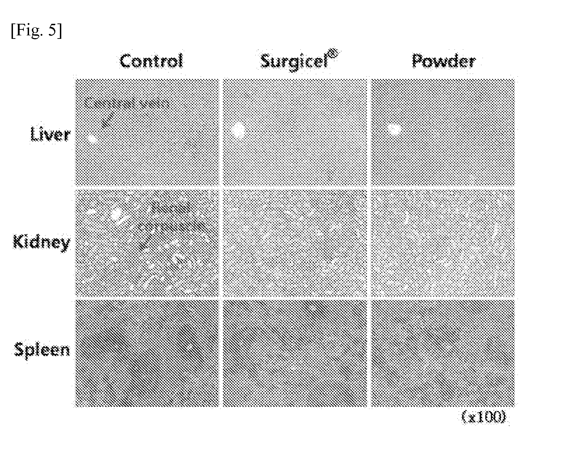

After confirming hemostasis of the fine particle hemostatic obtained in example 13 (described as 'powder' in Figs. 4 and 5) after the river of the 8weeks healthy SD-rat was cut, the remaining or not at the day after an operation and the inflammation or not at surrounding tissue after two weeks were confirmed. The negative control (described as 'control' in Figs. 4 and 5) was no treatment, and the positive control was used Surgicel that a hemostatic on the market.

As a result, the fine particle hemostatic had an effect of decreasing the blood loss when bleeding by promptly acting on the bleeding part, consequently the dead number was decreased. In addition, the day after an operation, as seen at Fig. 4, Surgicel 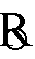 was not yet degraded and almost the whole was remained, meanwhile the fine particle hemostatic prepared according to example 13 was completely degraded to show the same tissue state as negative control.

Also, as a result of observing the surrounding tissue being treated with hemostatic after 2 weeks, Surgicel 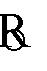 showed the severe adhesion degree perimetrically, meanwhile the fine particle hemostatic prepared according to example 13 showed adhesion prevention effect as well as promptly easy hemostasis as a hemostatic for inner use(at in-vivo operation).

As a result of analyzing the extracted organs after observation, as seen in Fig. 5, there was no allergy reaction such as infiltration of inflammation cell or necrosis even at fine particle hemostatic as the controls.

While the present invention has been described with respect to the specific embodiments, it will be apparent to those skilled in the art that various changes and modifications may be made without departing from the spirit and scope of the invention as defined in the following claims.

### Industrial Availability

The functional fine particle composition capable of spraying according to the present invention improves availability of treatment and narrowness of the treatment range, the possibility of contamination by contacting which are drawbacks of the gel, solution, sponge, fabric, aerosol types of hemostatic, improves inflammation and foreign response, the possibility of denaturing at degradation, particularity of the storage requirements, etc. which are drawbacks of oxycellulose, gelatin, protein agent-containing hemostatic based hemostatics. Accordingly, it is promptly and easy to treat to the wounded part, and shows the prompt hemostasis of the biocompatible-biodegradable polymer having the hemostasis performance, and the wounds protection and adhesion prevention effect remaining to be adhered to the wounds, for heat-sensitive functional polymer.

## Claims

1. A functional fine particle composition capable of spraying, comprising:
a) a double-layered fine particle comprising i) a core comprising heat-sensitive functional polymer and ii) an outer layer surrounding the core, comprising biodegradable-biocompatible polymer; and
b) a volatile carrier carrying the fine particle.

2. The composition of claim 1, wherein the heat-sensitive functional polymer of i) is supported with a drug.

3. The composition of claim 1, wherein the biodegradable-biocompatible polymer of ii) comprises at least one polymer selected from glycoaminoglycan selected from the group consisting of chondroitin sulfate, dermatan sulfate, keratan sulfate, heparan sulphate, hyaluronic acid, alginate and heparin; protein selected from the group consisting of collagen, gelatin, elastin and fibrin; proteoglycan selected from the group consisting of versican, aglycan, parcan, decorin, viglican, celgricin and syndecan; glycoprotein selected from the group consisting of fibronectin, laminin, vitronectin, thrombospondin and tenascin; phospholipid selected from the group consisting of phosphatidylcholine, phosphatidylserine, phosphatidylethanolamine, sphingomyelin and derivatives thereof; or glycolipid selected from the group consisting of cerebroside, ganglioside, gallactocerebroside and derivatives thereof, and cholesterol.

4. The composition of claim 1, wherein the outer layer is crosslinked by a crosslinker selected from the group consisting of a compound containing at least one cation selected from the group consisting of Mg²⁺, Mn²⁺, Ca²⁺, Co²⁺, Cu²⁺, Sr²⁺, Ba²⁺ and Fe²⁺; an acrylic polymer of chitosan, glutaraldehyde, formalin, poly-L-lysine, polyacrylic acid and polymethacrylic acid; a dopamine-containing hydroxylamine compound; amino acids of isoleucine, phenylalanine, leucine, threonine, lysine, tryptophan, methionine, valline, histidine, alanine, arginine, asparagine, aspartate, cysteine, glutamine, glutamate, glycine, proline, serine and tyrosine and polymer thereof.

5. The composition of claim 1, wherein the core comprises at least one copolymer selected from the consisting of polyethyleneglycol-polypropyleneglycol copolymer, polyethyleneglycol-polylactic acid copolymer, polyethyleneglycol-polylactic glycolic acid copolymer and polyethyleneglycol-polycaprolactone copolymer.

6. The composition of claim 5, wherein the copolymer has weight average molecular weight of 1,000dalton to 100,000dalton.

7. The composition of claim 1, wherein the core is supported with a drug and the drug is used by combining one or more selected from the group consisting of thrombin, aprotinin, steroidal anti-inflammatory drug and non-steroidal anti-inflammatory drug.

8. The composition of claim 7, wherein the core is supported with the drug in amount of 0.1 to 40% by weight.

9. The composition of claim 1, wherein the biodegradable-biocompatible polymer is in amount of 20 to 99% by weight, the heat-sensitive functional polymer is in amount of 1 to 80% by weight.

10. The composition of claim 1, wherein the double-layered fine particle has average particle diameter of 0.01 µm to 400 µm.

11. The composition of claim 1, wherein the volatile carrier is selected from the group consisting of ethanol, isopropanol, propanol, butanol, 1,1-fluoroethane, propane, butane, nitrogen, air and carbon dioxide.

12. The composition of claim 1, wherein the volatile carrier contains in amount of 20 to 90% by weight in the composition.

13. A method of preparing a functional fine particle composition capable of spraying, comprising:
a) forming a core of double-layered fine particle comprising heat-sensitive functional polymer and capable of supporting with drugs;
b) forming an outer layer of double-layered fine particle surrounding the core and comprising biodegradable-biocompatible polymer; and
c) filling a volatile carrier.

14. The method of claim 13, wherein the double-layered fine particle is prepared by a method selected from the group consisting of a) a method of preparing powder by forming an emulsion followed by drying, b) a method of preparing powder by extracting an emulsion to nonsolvent, c) a method of preparing by spray-coating to powder for core followed by drying, d) a method of preparing powder by mixing a biodegradable hemostatic polymer with heat-sensitive copolymer followed by spray-drying, e) a method of preparing power by electric spraying, f) a method of preparing fine particle by pulverizing dried powder of biodegradable hemostatic polymer and heat-sensitive copolymer mixture.

15. The method of claim 13, wherein the biodegradable-biocompatible polymer comprises at least one polymer selected from glycoaminoglycan selected from the group consisting of chondroitin sulfate, dermatan sulfate, keratan sulfate, heparan sulphate, hyaluronic acid, alginate and heparin; protein selected from the group consisting of collagen, gelatin, elastin and fibrin; proteoglycan selected from the group consisting of versican, aglycan, parcan, decorin, viglican, celgricin and syndecan; glycoprotein selected from the group consisting of fibronectin, laminin, vitronectin, thrombospondin and tenascin; phospholipid selected from the group consisting of phosphatidylcholine, phosphatidylserine, phosphatidylethanolamine, sphingomyelin and derivatives thereof; or glycolipid selected from the group consisting of cerebroside, ganglioside, gallactocerebroside and derivatives thereof, and cholesterol.

16. The method of claim 13, wherein the heat-sensitive functional polymer comprises at least one copolymer selected from the consisting of polyethyleneglycol-polypropyleneglycol copolymer, polyethyleneglycol-polylactic acid copolymer, polyethyleneglycol-polylactic glycolic acid copolymer and polyethyleneglycol-polycaprolactone copolymer.

17. The method of claim 13, wherein the outer layer is crosslinked by a crosslinker selected from the group consisting of a compound containing at least one cation selected from the group consisting of Mg²⁺, Mn²⁺, Ca²⁺, Co²⁺, Cu²⁺, Sr²⁺, Ba²⁺ and Fe²⁺, an acrylic polymer of chitosan, glutaraldehyde, formalin, poly-L-lysine, polyacrylic acid and polymethacrylic acid; a dopamine-containing hydroxylamine compound; amino acids of isoleucine, phenylalanine, leucine, threonine, lysine, tryptophan, methionine, valline, histidine, alanine, arginine, asparagine, aspartate, cysteine, glutamine, glutamate, glycine, proline, serine and tyrosine and polymer thereof.

18. The method of claim 13, wherein the drug of a) is used by combining one or more selected from the group consisting of thrombin, aprotinin, steroidal anti-inflammatory drug and non-steroidal anti-inflammatory drug.

19. The method of claim 13, wherein the volatile carrier of c) is selected from the group consisting of ethanol, isopropanol, propanol, butanol, 1,1-fluoroethane, liquefied propane, liquefied butane, nitrogen, air and carbon dioxide.

20. A sprayer comprising a functional fine particle composition of any one of claims 1 to 12.
